# EUROPEAN PATENT APPLICATION

(11) **EP 3 957 630 A1**
(43) Date of publication of application: **23.02.2022**
(21) Application number: 20191703.6
(22) Date of filing: 19.08.2020
(51) Int. Cl.: C07D 401/04, A01N 43/40

(54) **CRYSTALLINE MONOHYDRATE OF 4-AMINO-3-CHLORO-5-FLUORO-6-(7-FLUORO-1H-INDOL-6-YL)PICOLINIC ACID SUMMARY OF THE INVENTION**

(71) Applicant: Bayer Aktiengesellschaft, 51373 Leverkusen (DE)
(72) Inventor: OLENIK, Britta, 46242 Bottrop (DE); KEIL, Birgit, 40231 Düsseldorf (DE); RÖSLER, Bernd, 42115 Wuppertal (DE)
(74) Representative: BIP Patents

(57) **Abstract**

The present invention relates to a novel crystalline monohydrate of 4-amino-3-chloro-5-fluoro-6-(7-fluoro-1*H*-indol-6-yl)picolinic acid according to formula (1), 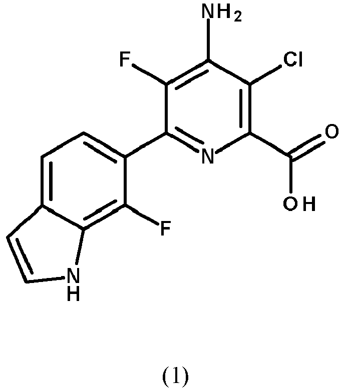 to a process for its preparation, to agrochemical formulations comprising said novel crystalline form, to its use for the for the production of a formulation with increased stability and to its use in plant protection applications, especially to its use as a herbicide.

## Description

### Summary of the invention

The present invention relates to a novel crystalline monohydrate of 4-amino-3-chloro-5-fluoro-6-(7-fluoro-1*H*-indol-6-yl)picolinic acid according to formula (1), to a process for its preparation, to agrochemical formulations comprising said novel crystalline form, to its use for the production of a formulation with increased stability and to its use in plant protection applications, especially to its use as a herbicide.

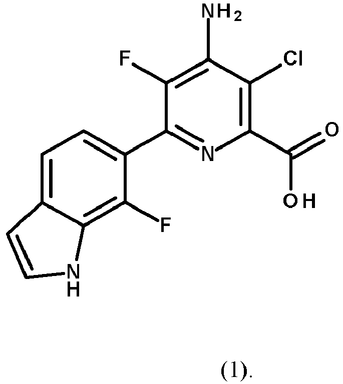

4-Amino-3-chloro-5-fluoro-6-(7-fluoro-1*H*-indol-6-yl)picolinic acid, which hereinafter is also termed as A4356716H, and processes for its production are known from WO2014/151005 and WO2018/208582.

### Background

Polymorphism is the ability of a compound to crystallize in different crystalline phases with different arrangements and/or conformations of the molecules in the crystal lattice. Hence, polymorphs are different crystalline forms of the same pure chemical compound. On account of the different arrangement and/or conformation of molecules, polymorphs exhibit different physical, chemical and biological properties. Properties which may be affected include but are not limited solubility, dissolution rate, stability, optical and mechanical properties, etc. The relative stability of a polymorph depends on its free energy, i.e. a more stable polymorph has a lower free energy. Under a defined set of experimental conditions only one polymorph has the lowest free energy. This polymorph is the thermodynamically stable form and all other polymorph(s) is (are) termed metastable form(s). A metastable form is one that is thermodynamically unstable but can nevertheless be prepared, isolated and analyzed as a result of its relatively slow rate of transformation.

The occurrence of active substances in different polymorphic forms (hereinafter also named as polymorphs or crystalline forms) is of decisive importance for the production in industrial scale as well as for the development of formulations containing the active substance, as unwanted phase change can lead to thickening and potentially solidification of the formulation and/or large crystals, which can lead to blockages in application equipment, e.g. in spray nozzles in agricultural application machinery. The knowledge of the existence of crystalline modifications and their properties is thus of high relevance. Each polymorph is characterized by a specific, uniform packing and arrangement of the molecules in the solid state. Nevertheless, it is generally not predictable whether a given chemical compound forms polymorphs at all and if so, which physical and biological properties the different polymorphs may have.

In addition pseudopolymophic forms, in particular hydrates or solvates, can occur. A solvate is a crystalline molecular compound in which molecules of the solvent of crystallisation are incorporated into the host lattice, consisting of unsolvated molecules. A hydrate is a special case of a solvate, when the incorporated solvent is water. The presence of solvent molecules in the crystal lattice influences the intermolecular interactions and confers unique physical properties to each solvate. A solvate thus has its own characteristic values of internal energy, enthalpy, entropy, Gibbs free energy, and thermodynamic activity.

### Figures

**Fig. 1****:** X-ray powder diffractogram of crystalline monohydrate of 4-amino-3-chloro-5-fluoro-6-(7-fluoro-1*H*-indol-6-yl)picolinic acid
**Fig. 2****:** X-ray powder diffractogram of crystalline dihydrate of 4-amino-3-chloro-5-fluoro-6-(7-fluoro-1*H*-indol-6-yl)picolinic acid
**Fig. 3****:** X-ray powder diffractogram of crystalline nonstoichiometric hydrate of 4-amino-3-chloro-5-fluoro-6-(7-fluoro-1*H*-indol-6-yl)picolinic acid

### Detailed description

In a first aspect, the present invention relates to a novel crystalline monohydrate of 4-amino-3-chloro-5-fluoro-6-(7-fluoro-1*H*-indol-6-yl)picolinic acid according to the following formula (1):

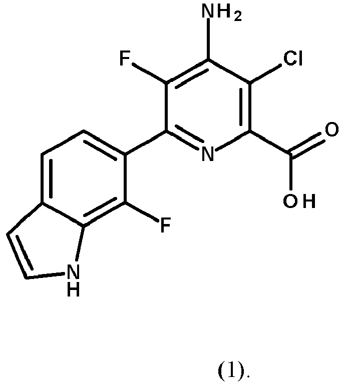

4-Amino-3-chloro-5-fluoro-6-(7-fluoro-1*H*-indol-6-yl)picolinic acid is also termed as A4356716H in the context of the present invention.

Attempts to convert A4356716H into a water-free crystalline form, which is typically the thermodynamic stable form, failed.

Object of the present invention is therefore the provision of a crystalline form of A4356716H of formula (1), preferably having superior application properties, beneficial physicochemical (e.g. stability) and/or formulation properties.

It has now surprisingly been found that the crystalline monohydrate, which is obtained by applying specific crystallisation conditions, has such desired and beneficial properties. In particular, the crystalline monohydrate displayed improved properties, *inter alia* concerning mechanical stability, in particular when compared with other hydrate forms of 4-amino-3-chloro-5-fluoro-6-(7-fluoro-1*H*-indol-6-yl)picolinic acid, in particular compared to the dihydrate and the nonstoichiometric hydrate described hereinbelow. In particular, the monohydrate displays a superior stability and thereby ensures that an undesired conversion into another hydrate form - such as the dihydrate and the nonstoichiometric hydrate described hereinbelow - of the compound of formula (1) take place, and thereby associated changes in the properties as described above are prevented. The superior stability enhances the quality of formulations comprising the monohydrate of the compound of formula (1).

It particular, it was found that the monohydrate of the compound of the formula (1) is sufficiently stable and robust to be used for (the production of) agrochemical formulations. Thus, based on the properties found for the monohydrate, it can be assumed that the monohydrate has sufficiently higher stability against conversion than the dihydrate and the nonstoichiometric hydrate also described herein.

The monohydrate has improved handling and formulation properties, in particular in view of the observed mechanical stability of said monohydrate, and the fact that said monohydrate does for example not convert into other hydrate forms described herein below during storage in a water containing formulation or a humid environment.

The monohydrate of 4-amino-3-chloro-5-fluoro-6-(7-fluoro-1*H*-indol-6-yl)picolinic acid can be characterized by X-ray powder diffractometry on the basis of the respective diffraction diagrams, which are recorded at 25°C and with Cu-Kα 1 radiation (1.5406 Å). All X-ray reflections are quoted as °2θ (theta) values (peak maxima) with a resolution of ±0.2°.

The monohydrate according to the present invention displays at least 3, often at least 5, in particular preferably at least 7, more preferably at least 10, and especially all of the reflections quoted in the following Table 1 as values, each quoted as 2θ value ± 0.2°.

**Table 1: X-ray reflections of monohydrate of A4356716H**

| Reflections [2θ values] x ± 0.2° | Reflections [2θ values] x ± 0.2° |
|---|---|
| 7.9 | 23.8 |
| 8.3 | 24.8 |
| 9.3 | 25.3 |
| 9.5 | 26.2 |
| 11.4 | 27.1 |
| 12.1 | 28.7 |
| 13.1 | 29.0 |
| 13.6 | 29.6 |
| 14.4 | 30.4 |
| 15.1 | 31.2 |
| 15.5 | 33.0 |
| 16.5 | 33.4 |
| 18.3 | 34.1 |
| 18.7 | 34.4 |
| 18.9 | 36.3 |
| 20.9 | 37.3 |
| 21.2 | 37.8 |
| 22.5 | 39.3 |
| 23.4 | |

The X-ray powder diffractogram at 25°C and Cu-Kα 1 of the monohydrate of A4356716H according to the present invention displays at least the following reflections: 24.8; 26.2 and 31.2, each quoted as 2θ value ± 0.2°.

The X-ray powder diffractogram at 25°C and Cu-Kα 1 of the monohydrate of A4356716H according to the present invention displays at least the following reflections: 24.8; 26.2; 31.2; 34.1 and 16.5, each quoted as 2θ value ± 0.2°.

The X-ray powder diffractogram at 25°C and Cu-Kα 1 of the monohydrate of A4356716H according to the present invention displays at least the following reflections: 24.8; 26.2; 31.2; 34.1; 16.5; 34.4 and 7.9, each quoted as 2θ value ± 0.2°.

The X-ray powder diffractogram at 25°C and Cu-Kα 1 of the monohydrate of A4356716H according to the present invention displays at least the following reflections: 24.8; 26.2; 31.2; 34.1; 16.5; 34.4; 7.9; 12.1 and 39.3, each quoted as 2θ value ± 0.2°.

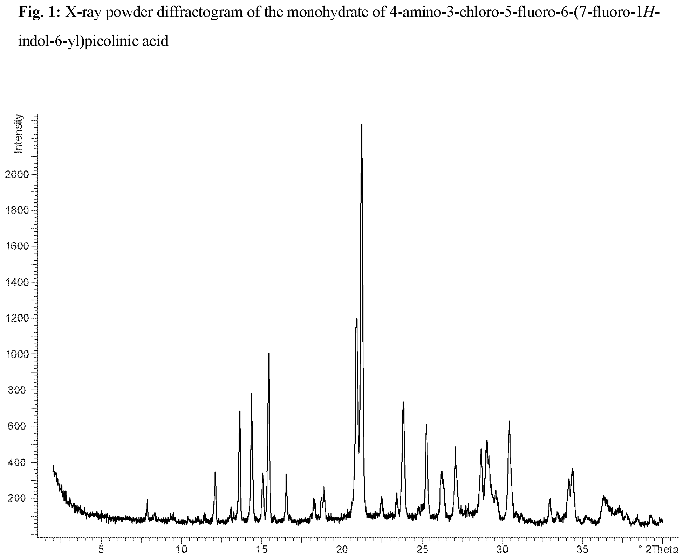

The monohydrate of A4356716H according to the present invention is further characterized by the X-ray powder diffractogram depicted in **Fig. 1****.**

In addition to the monohydrate, a dihydrate of 4-amino-3-chloro-5-fluoro-6-(7-fluoro-1*H*-indol-6-yl)picolinic acid and a nonstoichiometric hydrate of 4-amino-3-chloro-5-fluoro-6-(7-fluoro-1*H*-indol-6-yl)picolinic acid have been identified, which are further described in the following.

The dihydrate and the nonstoichiometric hydrate of 4-amino-3-chloro-5-fluoro-6-(7-fluoro-1*H*-indol-6-yl)picolinic acid can be characterized by X-ray powder diffractometry on the basis of the respective diffraction diagrams, which are recorded at 25°C and with Cu-Kα 1 radiation (1.5406 Å).

The dihydrate of A4356716H and the nonstoichiometric hydrate of A4356716H the reflections quoted in the following Table 2 as values:

**Table 2: X-ray reflections of the dihydrate of A4356716H ("Dihydrate") and of the nonstoichiometric hydrate of A4356716H ("Nonstoichiometric hydrate")**

| Reflections [2θ values] x±0.2° | |
|---|---|
| **Dihydrate** | **Nonstoichiometric hydrate** |
| 8.6 | 6.4 |
| 9.4 | 8.3 |
| 13.4 | 9.4 |
| 14.0 | 11.4 |

| Reflections [2θ values] x±0.2° | |
|---|---|
| **Dihydrate** | **Nonstoichiometric hydrate** |
| 15.2 | 12.7 |
| 17.3 | 13.2 |
| 18.4 | 14.8 |
| 18.7 | 16.2 |
| 21.3 | 17.4 |
| 22.6 | 19.8 |
| 23.4 | 21.2 |
| 23.8 | 22.1 |
| 25.1 | 23.1 |
| 25.9 | 24.0 |
| 26.6 | 25.7 |
| 26.9 | 26.5 |
| 27.9 | 26.9 |
| 28.2 | 27.4 |
| 28.5 | 27.7 |
| 28.8 | 28.5 |
| 29.8 | 29.0 |
| 30.5 | 29.9 |
| 30.9 | 30.1 |
| 31.6 | 30.5 |
| 33.0 | 32.7 |
| 33.5 | 33.0 |
| 34.9 | 33.5 |
| 35.4 | 35.3 |
| 36.5 | 36.2 |
| 37.2 | 37.6 |
| 38.7 | 38.1 |

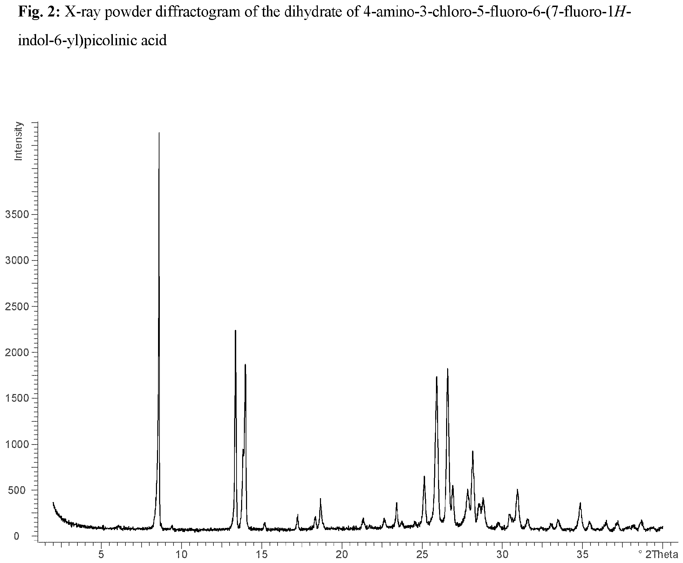

The dihydrate of A4356716H is further characterized by the X-ray powder diffractogram depicted in **Fig. 2****.**

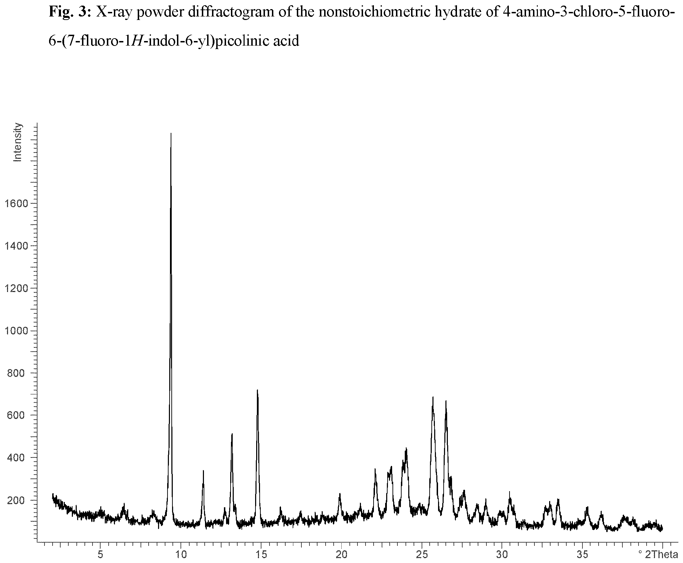

The nonstoichiometric hydrate of A4356716H is further characterized by the X-ray powder diffractogram depicted in **Fig. 3****.**

In a further aspect, the present invention is directed to a process for the production of the monohydrate according to the present invention, comprising the following steps:
a) suspending the compound of formula (1), preferably of the dihydrate, in a solvent or solvent mixture selected from the group consisting of aliphatic esters with a total of 3 to 6 carbon atoms;
b) stirring the suspension of step a) at a temperature in the range of from about 15°C to about 30°C for about 24 to 240 hours in a closed vessel;
c) evaporating the solvent or solvent mixture from the mixture obtained in step b) at a temperature in the range of from about 15°C to about 30°C;
d) isolating the precipitate obtained in step c) comprising or consisting of the monohydrate, optionally followed by subsequent drying the precipitate comprising or consisting of the monophydrate.

The chemical preparation of A4356716H according to formula (1) is known from WO2014/151005 and WO2018/208582. The compound of formula (1) as used in step a) can thus be prepared according to WO2014/151005 and WO2018/208582, to which full reference is made hereby.

The compound of formula (1) in step a) can essentially be any form of A4356716H. This means that A4356716H can be used in amorphous form, as a mixture of different pseudopolymorphic forms or as a mixture containing an amorphous and one or more different pseudopolymorphic forms.

Suitable solvents or solvent mixtures which can be used to dilute and/or suspend the compound of formula (1) in step a) and from which the compound of formula (1) is obtained as crystalline monohydrate, are selected from the group consisting of
(i) aliphatic esters with a total of 3 to 6 carbon atoms, wherein preferably the aliphatic esters are C₁-C₄-esters of acetic acid, and preferably selected from the group consisting of methyl acetate, ethyl acetate, n-propyl acetate or n-butyl acetate, and most preferably the solvent is ethyl acetate,
   or
(ii) isopropanol.

In step b) the suspension obtained in step a) is preferably stirred at a temperature in the range of about 18°C to about 25°C, typically for about 120 to 200 hours.

In step c) the solvent or solvent mixture from the mixture obtained in step b) is preferably evaporated at a temperature in the range of about 18°C to about 25°C at atmospheric pressure (about 1013 mbar).

Alternatively, the crystalline monohydrate can be isolated from the solvent or solvent mixture by allowing the suspension resulting from step b) until a substantial amount of the solvent, oftentimes about 90 wt.-% of the solvent or solvent mixture, has evaporated.

Typically, the precipitate obtained in step c) comprises or consists of the crystalline monohydrate which optionally may subsequently be dried according to usual methods known in the art.

The isolation of the crystalline monohydrate from the mother liquid may alternatively be effected by common techniques known in the art, for example by filtration, centrifugation or by decanting.

The crystallization of the crystalline monohydrate can be promoted or accelerated by seeding with seed crystals of the monohydrate.

The isolated crystalline monohydrate can optionally be washed with any solvent, preferably with the solvent or solvent mixture used for crystallization. The washing step can optionally be repeated, whereby washing is typically performed at temperatures below 30°C, often below 25°C.

In a further, optionally step, the crystals of the crystalline monohydrate can be dried and then supplied for further processing.

By means of the crystallization according to the present invention, the crystalline monohydrate of A4356716H is obtained with at least 85 %, in particular with at least 90 %, and most preferably with at least 95 %.

Thus, a particular embodiment of the present invention relates to 4-amino-3-chloro-5-fluoro-6-(7-fluoro-1*H-*indol-6-yl)picolinic acid, which consists of at least 85%, preferably of at least 90 % and particularly preferably of 95 % or more of the crystalline monohydrate.

In a further aspect, the present invention is directed to a plant protection agent in the form of customary formulations containing the crystalline monohydrate of 4-amino-3-chloro-5-fluoro-6-(7-fluoro-1*H*-indol-6-yl)picolinic acid.

The plant protection agent may additionally comprise one or more further active substance(s) selected from the group consisting of herbicides, insecticides, acaricides, fungicides, safeners and/or plant growth regulators.

Such suitable and preferred further active substance(s) are selected from the group of herbicides and safeners, more specifically the herbicides and the herbicide safeners mentioned in the following.

In some embodiments, the compounds, compositions, and methods provided herein are used in conjunction with one or more other herbicides to control a wider variety of undesirable vegetation. When used in conjunction with other herbicides, the crystalline monohydrate of 4-amino-3-chloro-5-fluoro-6-(7-fluoro-1*H-*indol-6-yl)picolinic acid of the present invention can be formulated with the other herbicide or herbicides, tank-mixed with the other herbicide or herbicides or applied sequentially with the other herbicide or herbicides. Some of the herbicides that can be employed in conjunction with the compounds of the present disclosure include: 4-CPA, 4-CPB, 4-CPP, 2,4-D, 2,4-D choline salt, 2,4-D esters and amines, 2,4-DB, 3,4-DA, 3,4-DB, 2,4-DEB, 2,4-DEP, 3,4-DP, 2,3,6-TBA, 2,4,5-T, 2,4,5-TB, acetochlor, acifluorfen, aclonifen, acrolein, alachlor, allidochlor, alloxydim, allyl alcohol, alorac, ametridione, ametryn, amibuzin, amicarbazone, amidosulfuron, aminocyclopyrachlor, aminopyralid, amiprofos-methyl, amitrole, ammonium sulfamate, anilofos, anisuron, asulam, atraton, atrazine, azafenidin, azimsulfuron, aziprotryne, barban, BCPC, beflubutamid, benazolin, bencarbazone, benfluralin, benfuresate, bensulfuron-methyl, bensulide, benthiocarb, bentazon-sodium, benzadox, benzfendizone, benzipram, benzobicyclon, benzofenap, benzofluor, benzoylprop, benzthiazuron, bicyclopyrone, bifenox, bilanafos, bispyribac-sodium, borax, bromacil, bromobonil, bromobutide, bromofenoxim, bromoxynil, brompyrazon, butachlor, butafenacil, butamifos, butenachlor, buthidazole, buthiuron, butralin, butroxydim, buturon, butylate, cacodylic acid, cafenstrole, calcium chlorate, calcium cyanamide, cambendichlor, carbasulam, carbetamide, carboxazole, chlorprocarb, carfentrazone-ethyl, CDEA, CEPC, chlomethoxyfen, chloramben, chloranocryl, chlorazifop, chlorazine, chlorbromuron, chlorbufam, chloreturon, chlorfenac, chlorfenprop, chlorflurazole, chlorflurenol, chloridazon, chlorimuron, chlornitrofen, chloropon, chlorotoluron, chloroxuron, chloroxynil, chlorpropham, chlorsulfuron, chlorthal, chlorthiamid, cinidon-ethyl, cinmethylin, cinosulfuron, cisanilide, clethodim, cliodinate, clodinafop-propargyl, clofop, clomazone, clomeprop, cloprop, cloproxydim, clopyralid, cloransulam-methyl, CMA, copper sulfate, CPMF, CPPC, credazine, cresol, cumyluron, cyanatryn, cyanazine, cycloate, cyclosulfamuron, cycloxydim, cycluron, cyhalofop-butyl, cyperquat, cyprazine, cyprazole, cypromid, daimuron, dalapon, dazomet, delachlor, desmedipham, desmetryn, di-allate, dicamba, dichlobenil, dichloralurea, dichlormate, dichlorprop, dichlorprop-P, diclofop, diclosulam, diethamquat, diethatyl, difenopenten, difenoxuron, difenzoquat, diflufenican, diflufenzopyr, dimefuron, dimepiperate, dimethachlor, dimethametryn, dimethenamid, dimethenamid-P, dimexano, dimidazon, dinitramine, dinofenate, dinoprop, dinosam, dinoseb, dinoterb, diphenamid, dipropetryn, diquat, disul, dithiopyr, diuron, DMPA, DNOC, DSMA, EBEP, eglinazine, endothal, epronaz, EPTC, erbon, esprocarb, ethalfluralin, ethbenzamide, ethametsulfuron, ethidimuron, ethiolate, ethobenzamid, etobenzamid, ethofumesate, ethoxyfen, ethoxysulfuron, etinofen, etnipromid, etobenzanid, EXD, fenasulam, fenoprop, fenoxaprop, fenoxaprop-P-ethyl, fenoxaprop-P-ethyl + isoxadifen-ethyl, fenoxasulfone, fenteracol, fenthiaprop, fentrazamide, fenuron, ferrous sulfate, flamprop, flamprop-M, flazasulfuron, florasulam, fluazifop, fluazifop-P-butyl, fluazolate, flucarbazone, flucetosulfuron, fluchloralin, flufenacet, flufenican, flufenpyr-ethyl, flumetsulam, flumezin, flumiclorac-pentyl, flumioxazin, flumipropyn, fluometuron, fluorodifen, fluoroglycofen, fluoromidine, fluoronitrofen, fluothiuron, flupoxam, flupropacil, flupropanate, flupyrsulfuron, fluridone, flurochloridone, fluroxypyr, flurtamone, fluthiacet, fomesafen, foramsulfuron, fosamine, furyloxyfen, glufosinate, glufosinate-ammonium, glyphosate, halosafen, halosulfuron-methyl, haloxydine, haloxyfop-methyl, haloxyfop-P-methyl, halauxifen-methyl, hexachloroacetone, hexaflurate, hexazinone, imazamethabenz, imazamox, imazapic, imazapyr, imazaquin, imazethapyr, imazosulfuron, indanofan, indaziflam, iodobonil, iodomethane, iodosulfuron, iofensulfuron, ioxynil, ipazine, ipfencarbazone, iprymidam, isocarbamid, isocil, isomethiozin, isonoruron, isopolinate, isopropalin, isoproturon, isouron, isoxaben, isoxachlortole, isoxaflutole, isoxapyrifop, karbutilate, ketospiradox, lactofen, lenacil, linuron, MAA, MAMA, MCPA esters and amines, MCPA-thioethyl, MCPB, mecoprop, mecoprop-P, medinoterb, mefenacet, mefluidide, mesoprazine, mesosulfuron, mesotrione, metam, metamifop, metamitron, metazachlor, metazosulfuron, metflurazon, methabenzthiazuron, methalpropalin, methazole, methiobencarb, methiozolin, methiuron, methometon, methoprotryne, methyl bromide, methyl isothiocyanate, methyldymron, metobenzuron, metobromuron, metolachlor, metosulam, metoxuron, metribuzin, metsulfuron, molinate, monalide, monisouron, monochloroacetic acid, monolinuron, monuron, morfamquat, MSMA, naproanilide, napropamide, napropamide-M, naptalam, neburon, nicosulfuron, nipyraclofen, nitralin, nitrofen, nitrofluorfen, norflurazon, noruron, OCH, orbencarb, ortho-dichlorobenzene, orthosulfamuron, oryzalin, oxadiargyl, oxadiazon, oxapyrazon, oxasulfuron, oxaziclomefone, oxyfluorfen, paraflufen-ethyl, parafluron, paraquat, pebulate, pelargonic acid, pendimethalin, penoxsulam, pentachlorophenol, pentanochlor, pentoxazone, perfluidone, pethoxamid, phenisopham, phenmedipham, phenmedipham-ethyl, phenobenzuron, phenylmercury acetate, picloram, picolinafen, pinoxaden, piperophos, potassium arsenite, potassium azide, potassium cyanate, pretilachlor, primisulfuron-methyl, procyazine, prodiamine, profluazol, profluralin, profoxydim, proglinazine, prohexadione-calcium, prometon, prometryn, propachlor, propanil, propaquizafop, propazine, propham, propisochlor, propoxycarbazone, propyrisulfuron, propyzamide, prosulfalin, prosulfocarb, prosulfuron, proxan, prynachlor, pydanon, pyraclonil, pyraflufen, pyrasulfotole, pyrazogyl, pyrazolynate, pyrazosulfuron-ethyl, pyrazoxyfen, pyribenzoxim, pyributicarb, pyriclor, pyridafol, pyridate, pyriftalid, pynminobac, pyrimisulfan, pyrithiobac-methyl, pyroxasulfone, pyroxsulam, quinclorac, quinmerac, quinoclamine, quinonamid, quizalofop, quizalofop-P-ethyl, rhodethanil, rimsulfuron, saflufenacil, S-metolachlor, sebuthylazine, secbumeton, sethoxydim, siduron, simazine, simeton, simetryn, SMA, sodium arsenite, sodium azide, sodium chlorate, sulcotrione, sulfallate, sulfentrazone, sulfometuron, sulfosate, sulfosulfuron, sulfuric acid, sulglycapin, swep, TCA, tebutam, tebuthiuron, tefuryltrione, tembotrione, tepraloxydim, terbacil, terbucarb, terbuchlor, terbumeton, terbuthylazine, terbutryn, tetrafluron, thenylchlor, thiazafluron, thiazopyr, thidiazimin, thidiazuron, thiencarbazone-methyl, thifensulfuron, thiobencarb, tiocarbazil, tioclorim, topramezone, tralkoxydim, triafamone, triallate, triasulfuron, triaziflam, tribenuron, tricamba, triclopyr esters and amines, tridiphane, trietazine, trifloxysulfuron, trifluralin, triflusulfuron, trifop, trifopsime, trihydroxytriazine, trimeturon, tripropindan, tritac, tritosulfuron, vernolate and xylachlor.

The crystalline monohydrate of 4-amino-3-chloro-5-fluoro-6-(7-fluoro-1*H*-indol-6-yl)picolinic acid of the present invention can generally be employed in combination with known herbicide safeners, such as benoxacor, benthiocarb, brassinolide, cloquintocet (e.g., mexyl), cyometrinil, daimuron, dichlormid, dicyclonon, dimepiperate, disulfoton, fenchlorazole-ethyl, fenclorim, flurazole, fluxofenim, furilazole, harpin proteins, isoxadifen-ethyl, mefenpyr-diethyl, cyprosulfamide, MG 191, MON 4660, naphthalic anhydride (NA), oxabetrinil, R29148 and N-phenylsulfonylbenzoic acid amides, to enhance their selectivity.

The plant protection agent may further comprise adjuvants which improve action, such as penetrants, e.g. vegetable oils, for example rapeseed oil, sunflower oil, mineral oils, for example paraffin oils, alkyl esters of vegetable fatty acids, for example rapeseed oil methyl ester or soya oil methyl ester, or alkanol alkoxylates and/or spreaders, for example alkylsiloxanes and/or salts, for example organic or inorganic ammonium or phosphonium salts, for example ammonium sulfate or diammonium hydrogenphosphate and/or retention promoters, for example dioctyl sulfosuccinate or hydroxypropylguar polymers and/or humectants, for example glycerol and/or fertilizers, for example ammonium-, potassium- or phosphorus-containing fertilizers.

Customary formulations are, for example, water-soluble liquids (SL), emulsion concentrates (EC), emulsions in water (EW), suspension concentrates (SC, SE, FS, OD), water-dispersible granules (WG), granules (GR) and capsule concentrates (CS); these and further possible formulation types are described, for example, by Crop Life International and in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576.

It was surprisingly found that the crystalline monohydrate the compound of the formula (1) has improved handling and formulation properties, in particular in view of the observed mechanical stability of said crystalline monohydrate, and the fact that said crystalline monohydrate is and is for example not converted to other pseudopolymorphic hydrate forms during storage in a water containing formulation or a humid environment.

In a further embodiment, the present invention is therefore directed to (agrochemical) formulations containing the crystalline monohydrate of the present invention and water. Said aqueous (agrochemical) formulations preferably are in the form of suspension concentrates (SC, SE, FS, OD).

Further, the present invention is further directed to the use of the crystalline monohydrate of the compound of formula (1) for the production of a formulation, preferably an agrochemical formulation, wherein said (agrochemical) formulation exhibits improved (i) chemical stability of the compound of formula (1) and/or (ii) physical stability, thus resulting in an improved storage stability of said (agrochemical) formulation.

Preference is given to formulations or use forms comprising auxiliaries, for example extenders, solvents, spontaneity promoters, carriers, emulsifiers, dispersants, frost protection agents, biocides, thickeners and/or further auxiliaries, for example adjuvants. An adjuvant in this context is a component which enhances the biological effect of the formulation, without the component itself having any biological effect. Examples of adjuvants are agents which promote retention, spreading, attachment to the leaf surface or penetration.

These formulations are prepared in a known way, for example by mixing the crystalline monohydrate of the compound of the formula (1) with auxiliaries such as, for example, extenders, solvents and/or solid carriers and/or other auxiliaries such as, for example, surfactants. The formulations are produced either in suitable facilities or else before or during application.

The auxiliaries used may be substances suitable for imparting special properties, such as certain physical, technical and/or biological properties, to the formulation of the crystalline monohydrate of the compound of the formula (1), or to the use forms prepared from these formulations (for example ready-to-use pesticides such as spray liquors or seed dressing products).

Suitable extenders are, for example, water, polar and nonpolar organic chemical liquids, for example from the classes of the aromatic and non-aromatic hydrocarbons (such as paraffins, alkylbenzenes, alkylnaphthalenes, chlorobenzenes), the alcohols and polyols (which, if appropriate, may also be substituted, etherified and/or esterified), the ketones (such as acetone, cyclohexanone), esters (including fats and oils) and (poly)ethers, the unsubstituted and substituted amines, amides, lactams (such as N-alkylpyrrolidones) and lactones, the sulfones and sulfoxides (such as dimethyl sulfoxide).

If the extender utilized is water, it is also possible to use, for example, organic solvents as auxiliary solvents. Useful liquid solvents are essentially: aromatics such as xylene, toluene or alkylnaphthalenes, chlorinated aromatics or chlorinated aliphatic hydrocarbons such as chlorobenzenes, chloroethylenes or methylene chloride, aliphatic hydrocarbons such as cyclohexane or paraffins, for example mineral oil fractions, mineral and vegetable oils, alcohols such as butanol or glycol and their ethers and esters, ketones such as acetone, methyl ethyl ketone, methyl isobutyl ketone or cyclohexanone, strongly polar solvents such as dimethylformamide and dimethyl sulfoxide, and water.

In principle, it is possible to use all suitable solvents. Examples of suitable solvents are aromatic hydrocarbons, such as xylene, toluene or alkylnaphthalenes, chlorinated aromatic or aliphatic hydrocarbons, such as chlorobenzene, chloroethylene or methylene chloride, aliphatic hydrocarbons, such as cyclohexane, paraffins, mineral oil fractions, mineral and vegetable oils, alcohols, such as methanol, ethanol, isopropanol, butanol or glycol and their ethers and esters, ketones such as acetone, methyl ethyl ketone, methyl isobutyl ketone or cyclohexanone, strongly polar solvents, such as dimethyl sulfoxide, and also water.

In principle, it is possible to use all suitable carriers. Useful carriers especially include: for example ammonium salts and ground natural minerals such as kaolins, clays, talc, chalk, quartz, attapulgite, montmorillonite or diatomaceous earth, and ground synthetic minerals such as finely divided silica, alumina and natural or synthetic silicates, resins, waxes and/or solid fertilizers. It is likewise possible to use mixtures of such carriers. Useful carriers for granules include: for example crushed and fractionated natural rocks such as calcite, marble, pumice, sepiolite, dolomite, and synthetic granules of inorganic and organic flours, and also granules of organic material such as sawdust, paper, coconut shells, corn cobs and tobacco stalks.

It is also possible to use liquefied gaseous extenders or solvents. Especially suitable are those extenders or carriers which are gaseous at standard temperature and under atmospheric pressure, for example aerosol propellants such as halogenated hydrocarbons, and also butane, propane, nitrogen and carbon dioxide.

Examples of emulsifiers and/or foam formers, dispersants or wetting agents having ionic or nonionic properties or mixtures of these surfactants are salts of polyacrylic acid, salts of lignosulfonic acid, salts of phenolsulfonic acid or naphthalenesulfonic acid, polycondensates of ethylene oxide with fatty alcohols or with fatty acids or with fatty amines, with substituted phenols (preferably alkylphenols or arylphenols), salts of sulfosuccinic esters, taurine derivatives (preferably alkyl taurates), phosphoric esters of polyethoxylated alcohols or phenols, fatty acid esters of polyols, and derivatives of the compounds containing sulfates, sulfonates and phosphates, for example alkylaryl polyglycol ethers, alkylsulfonates, alkyl sulfates, arylsulfonates, protein hydrolyzates, lignosulfite waste liquors and methylcellulose. The presence of a surfactant is advantageous if the crystalline monohydrate of the compound of the formula (1) and/or one of the inert carriers is insoluble in water and when the application takes place in water.

Further auxiliaries which may be present in the formulations and the use forms derived therefrom are dyes such as inorganic pigments, for example iron oxide, titanium oxide and Prussian Blue, and organic dyes such as alizarin dyes, azo dyes and metal phthalocyanine dyes, and nutrients and trace nutrients such as salts of iron, manganese, boron, copper, cobalt, molybdenum and zinc.

Additional components which may be present are stabilizers, such as cold stabilizers, preservatives, antioxidants, light stabilizers, or other agents which improve chemical and/or physical stability. Foam generators or antifoams may also be present.

In addition, the formulations and the use forms derived therefrom may also comprise, as additional auxiliaries, stickers such as carboxymethylcellulose and natural and synthetic polymers in the form of powders, granules or latices, such as gum arabic, polyvinyl alcohol and polyvinyl acetate, or else natural phospholipids such as cephalins and lecithins and synthetic phospholipids. Further auxiliaries may be mineral and vegetable oils.

It is possible if appropriate for still further auxiliaries to be present in the formulations and the use forms derived therefrom. Examples of such additives are fragrances, protective colloids, binders, adhesives, thickeners, thixotropic agents, penetrants, retention promoters, stabilizers, sequestrants, complexing agents, humectants, spreaders. In general, the crystalline monohydrate of the compound of the formula (1) can be combined with any solid or liquid additive commonly used for formulation purposes.

Useful retention promoters include all those substances which reduce dynamic surface tension, for example dioctyl sulfosuccinate, or increase viscoelasticity, for example hydroxypropylguar polymers.

Suitable penetrants in the present context are all those substances which are usually used for improving the penetration of agrochemical active compounds into plants. Penetrants are defined in this context by their ability to penetrate from the (generally aqueous) application liquor and/or from the spray coating into the cuticle of the plant and hence increase the mobility of the active compounds in the cuticle. The method described in the literature (Baur et al., 1997, Pesticide Science 51, 131-152) can be used for determining this property. Examples include alcohol alkoxylates such as coconut fatty ethoxylate (10) or isotridecyl ethoxylate (12), fatty acid esters, for example rapeseed oil methyl ester or soya oil methyl ester, fatty amine alkoxylates, for example tallowamine ethoxylate (15), or ammonium and/or phosphonium salts, for example ammonium sulfate or diammonium hydrogenphosphate.

In a preferred embodiment of the present invention, the plant protection agent contains more than 90 wt.-%, and preferably more than 95 wt.-%, of the crystalline monohydrate of the compound of the formula (1) based on the total amount of all forms of the compound of the formula (1) present in the composition.

The (agrochemical) formulations of the present invention typically comprise between 0.001% and 90% by weight of the crystalline monohydrate of the compound of the formula (1), more preferably between 0.1% and 80% by weight of the crystalline monohydrate of the the compound of the formula (1), most preferably between 0.5% and 60% by weight of the crystalline monohydrate of the compound of the formula (1), in each case based on the total weight of the (agrochemical) formulation.

The content of the crystalline monohydrate of the compound of the formula (1) in the use forms, i.e. the forms intended for application to e.g. undesired plants, prepared from the (agrochemical) formulations may vary within wide ranges. The concentration of the crystalline monohydrate of the compound of the formula (1) in the use forms may typically be between 0.000001% and 5% by weight of the crystalline monohydrate of the compound of the formula (1), preferably between 0.00001% and 1% by weight, based on the total weight of the use form. Application is accomplished in a customary manner appropriate for the use forms.

In a further embodiment, the present invention is directed to the use of the crystalline monohydrate of the compound of formula (1) for combatting undesired plant growth. In a particular embodiment, the present invention is directed to combatting undesired plant growth in crops of useful plants. In a particular embodiment of the present invention, the useful plants are transgenic plants.

In a further embodiment, the present invention is directed to a method for combatting undesired plant growth, wherein the crystalline monohydrate or a plant protection agent as defined above containing the crystalline monohydrate , is applied to plants or the site of the undesired vegetation.

In some embodiments, the compounds and compositions described herein are applied as a post-emergence application, pre-emergence application, in-water application to flooded paddy rice or water bodies (e.g., ponds, lakes and streams), or burn-down application.

In some embodiments, the crystalline monohydrate or a plant protection agent as defined above containing the crystalline monohydrate described herein are utilized to control weeds in crops, including but not limited to citrus, apple, rubber, oil, palm, forestry, direct-seeded, water-seeded and transplanted rice, wheat, barley, oats, rye, sorghum, corn/maize, pastures, grasslands, rangelands, fallowland, turf, tree and vine orchards, aquatics, or row-crops, as well as non-crop settings, e.g., industrial vegetation management (IVM) or rights-of-way. In some embodiments, the compounds and compositions are used to control woody plants, broadleaf and grass weeds, or sedges.

In some embodiments, the crystalline monohydrate or a plant protection agent as defined above containing the crystalline monohydrate described herein are utilized to control undesirable vegetation in rice. In certain embodiments, the undesirable vegetation is Brachiaria platyphylla (Groseb.) Nash (broadleaf signalgrass, BRAPP), Digitaria sanguinalis (L.) Scop. (large crabgrass, DIGSA), Echinochloa crus-galli (L.) P. Beauv. (barnyardgrass, ECHCG), Echinochloa colonum (L.) LINK (junglerice, ECHCO), Echinochloa oryzoides (Ard.) Fritsch (early watergrass, ECHOR), Echinochloa oryzicola (Vasinger) Vasinger (late watergrass, ECHPH), Ischaemum rugosum Salisb. (saramollagrass, ISCRU), Leptochloa chinensis (L.) Nees (Chinese sprangletop, LEFCH), Leptochloa fascicularis (Lam.) Gray (bearded sprangletop, LEFFA), Leptochloa panicoides (Presl.) Hitchc. (Amazon sprangletop, LEFPA), Panicum dichotomiflorum (L.) Michx. (fall panicum, PANDI), Paspalum dilatatum Poir. (dallisgrass, PASDI), Cyperus difformis L. (smallflower flatsedge, CYPDI), Cyperus esculentus L. (yellow nutsedge, CYPES), Cyperus iria L. (rice flatsedge, CYPIR), Cyperus rotundus L. (purple nutsedge, CYPRO), Eleocharis species (ELOSS), Fimbristylis miliacea (L.) Vah1 (globe fringerush, FIMMI), Schoenoplectus juncoides Roxb. (Japanese bulrush, SPCJU), Schoenoplectus maritimus L. (sea clubrush, SCPMA), Schoenoplectus mucronatus L. (ricefield bulrush, SCPMU), Aeschynomene species, (jointvetch, AESSS), Alternanthera philoxeroides (Mart.) Griseb. (alligatorweed, ALRPH), Alisma plantago-aquatica L. (common waterplantain, ALSPA), Amaranthus species, (pigweeds and amaranths, AMASS), Ammannia coccinea Rottb. (redstem, AMMCO), Eclipta alba (L.) Hassk. (American false daisy, ECLAL), Heteranthera limosa (SW.) Willd./Vah1 (ducksalad, HETLI), Heteranthera reniformis R. & P. (roundleaf mudplantain, HETRE), Ipomoea hederacea (L.) Jacq. (ivyleaf morningglory, IPOHE), Lindernia dubia (L.) Pennell (low false pimpernel, LIDDU), Monochoria korsakowii Regel & Maack (monochoria, MOOKO), Monochoria vaginalis (Burm. F.) C. Presl ex Kuhth, (monochoria, MOOVA), Murdannia nudiflora (L.) Brenan (doveweed, MUDNU), Polygonum pensylvanicum L., (Pennsylvania smartweed, POLPY), Polygonum persicaria L. (ladysthumb, POLPE), Polygonum hydropiperoides Michx. (POLHP, mild smartweed), Rotala indica (Willd.) Koehne (Indian toothcup, ROTIN), Sagittaria species, (arrowhead, SAGSS), Sesbania exaltata (Raf.) Cory/Rydb. Ex Hill (hemp sesbania, SEBEX), or Sphenoclea zeylanica Gaertn. (gooseweed, SPDZE).

In some embodiments, the crystalline monohydrate or a plant protection agent as defined above containing the crystalline monohydrate described herein are utilized to control undesirable vegetation in cereals. In certain embodiments, the undesirable vegetation is Alopecurus myosuroides Huds. (blackgrass, ALOMY), Apera spica-venti (L.) Beauv. (windgrass, APESV), Avena fatua L. (wild oat, AVEFA), Bromus tectorum L. (downy brome, BROTE), Lolium multiflorum Lam. (Italian ryegrass, LOLMU), Phalaris minor Retz. (littleseed canarygrass, PHAMI), Poa annua L. (annual bluegrass, POANN), Setaria pumila (Poir.) Roemer & J.A. Schultes (yellow foxtail, SETPU), Setaria viridis (L.) Beauv. (green foxtail, SETVI), Cirsium arvense (L.) Scop. (Canada thistle, CIRAR), Galium aparine L. (catchweed bedstraw, GALAP), Kochia scoparia (L.) Schrad. (kochia, KCHSC), Lamium purpureum L. (purple deadnettle , LAMPU), Matricaria recutita L. (wild chamomile, MATCH), Matricaria matricarioides (Less.) Porter (pineappleweed, MATMT), Papaver rhoeas L. (common poppy, PAPRH), Polygonum convolvulus L. (wild buckwheat, POLCO), Salsola tragus L. (Russian thistle, SASKR), Stellaria media (L.) Vill. (common chickweed, STEME), Veronica persica Poir. (Persian speedwell, VERPE), Viola arvensis Murr. (field violet, VIOAR), or Viola tricolor L. (wild violet, VIOTR).

In some embodiments, the crystalline monohydrate or a plant protection agent as defined above containing the crystalline monohydrate described herein are utilized to control undesirable vegetation in range and pasture. In certain embodiments, the undesirable vegetation is Ambrosia artemisiifolia L. (common ragweed, AMBEL), Cassia obtusifolia (sickle pod, CASOB), Centaurea maculosa auct. non Lam. (spotted knapweed, CENMA), Cirsium arvense (L.) Scop. (Canada thistle, CIRAR), Convolvulus arvensis L. (field bindweed, CONAR), Euphorbia esula L. (leafy spurge, EPHES), Lactuca serriola L./Torn. (prickly lettuce, LACSE), Plantago lanceolata L. (buckhorn plantain, PLALA), Rumex obtusifolius L. (broadleaf dock, RUMOB), Sida spinosa L. (prickly sida, SIDSP), Sinapis arvensis L. (wild mustard, SINAR), Sonchus arvensis L. (perennial sowthistle, SONAR), Solidago species (goldenrod, SOOSS), Taraxacum officinale G.H. Weber ex Wiggers (dandelion, TAROF), Trifolium repens L. (white clover, TRFRE), or Urtica dioica L. (common nettle, URTDI).

In some embodiments, the crystalline monohydrate or a plant protection agent as defined above containing the crystalline monohydrate described herein are utilized to control undesirable vegetation found in row crops. In certain embodiments, the undesirable vegetation is Alopecurus myosuroides Huds. (blackgrass, ALOMY), Avena fatua (L.) (wild oat, AVEFA), Brachiaria platyphylla (Groseb.) Nash (broadleaf signalgrass, BRAPP), Digitaria sanguinalis (L.) Scop. (large crabgrass, DIGSA), Echinochloa crus-galli (L.) P. Beauv. (barnyardgrass, ECHCG), Echinochloa colonum (L.) Link (junglerice, ECHCO), Lolium multiflorum Lam. (Italian ryegrass, LOLMU), Panicum dichotomiflorum Michx. (fall panicum, PANDI), Panicum miliaceum L. (wild-proso millet, PANMI), Setaria faberi Herrm. (giant foxtail, SETFA), Setaria viridis (L.) Beauv. (green foxtail, SETVI), Sorghum halepense (L.) Pers. (Johnsongrass, SORHA), Sorghum bicolor (L.) Moench ssp. Arundinaceum (shattercane, SORVU), Cyperus esculentus L. (yellow nutsedge, CYPES), Cyperus rotundus L. (purple nutsedge, CYPRO), Abutilon theophrasti Medik. (velvetleaf, ABUTH), Amaranthus species (pigweeds and amaranths, AMASS), Ambrosia artemisiifolia L. (common ragweed, AMBEL), Ambrosia psilostachya DC. (western ragweed, AMBPS), Ambrosia trifida L. (giant ragweed, AMBTR), Asclepias syriaca L. (common milkweed, ASCSY), Chenopodium album L. (common lambsquarters, CHEAL), Cirsium arvense (L.) Scop. (Canada thistle, CIRAR), Commelina benghalensis L. (tropical spiderwort, COMBE), Datura stramonium L. (jimsonweed, DATST), Daucus carota L. (wild carrot, DAUCA), Euphorbia heterophylla L. (wild poinsettia, EPHHL), Erigeron bonariensis L. (hairy fleabane, ERIBO), Erigeron canadensis L. (Canadian fleabane, ERICA), Helianthus annuus L. (common sunflower, HELAN), Jacquemontia tamnifolia (L.) Griseb. (smallflower morningglory, IAQTA), Ipomoea hederacea (L.) Jacq. (ivyleaf morningglory, IPOHE), Ipomoea lacunosa L. (white morningglory, IPOLA), Lactuca serriola L./Torn. (prickly lettuce, LACSE), Portulaca oleracea L. (common purslane, POROL), Sida spinosa L. (prickly sida, SIDSP), Sinapis arvensis L. (wild mustard, SINAR), Solanum ptychanthum Dunal (eastern black nightshade, SOLPT), or Xanthium strumarium L. (common cocklebur, XANST).

### Working examples

### Methods

All data which is part of the present application has been prepared according to the methods described below unless otherwise indicated. The samples used for measurement were directly used and did not undergo any further sample preparation.

### X-ray powder diffraction (XRPD)

X-ray diffraction patterns were recorded at ambient temperature using XRD-diffractometers X Pert PRO (PANalytical) and STOE STADI-P (radiation Cu-Kα 1, wavelength 1.5406 Å). All X-ray reflections are quoted as °2θ (theta) values (peak maxima) with a resolution of ±0.2°.

### A Crystallizing experiments of 4-amino-3-chloro-5-fluoro-6-(7-fluoro-1H-indol-6-yl)picolinic acid

### Examples A1 to A9

500 mg of dihydrate (obtained as described in Example 2.1 below) were dissolved in about 40 mL the respective solvent at boiling temperature. The resulting solutions were filtered, an divided in 4 separate portions of about 15 mL each. The first portion was stored at ambient temperature (approximately 25°C), the second portion was stored in a refrigerator (4°C) and the third portion was stored in a freezer (-20°C). During storage, typically a substantial amount of the respective solvent evaporated.

To the fourth portion water (referred to as "+ Water" in Table A below) or n-heptane (referred to as "+ n-Heptane" in Table A below) was added at ambient temperature (approximately 25°C) until precipitation occurred.

The precipitate of each portion was filtered off after six days and dried at ambient temperature and ambient humidity.

The obtained solids were isolated and analyzed by X-ray powder diffraction (XRPD). The respective result is stated in column "Result" in Table A.

**Table A: Solvents and conditions used for crystallizing experiments**

| **Example** | **Solvent/Solvent Mixture** | **Temperature [°C]** | **Result** |
|---|---|---|---|
| A1 | THF | 25 | Dihydrate |
| A2 | THF | 4 | Nonstoichiometric hydrate |
| A3 | THF | -20 | Nonstoichiometric hydrate |
| A4 | THF + Water | 25 | Mixture of Dihydrate and Nonstoichiometric hydrate |
| A5 | Acetone | 25 | Dihydrate |
| A6 | Acetone | 4 | Mixture of Dihydrate and Nonstoichiometric hydrate |
| A7 | Acetone + n-Heptane | 25 | Amorphous form |
| A8 | Isopropanol | 4 | Monohydrate |
| A9 | Isopropanol | -20 | Monohydrate |

### Example I - Monohydrate of 4-amino-3-chloro-5-fluoro-6-(7-fluoro-1H-indol-6-yl)picolinic acid

### I.1 Preparation of the monohydrate of A4356716H of formula (1)

### Example 1.1

100 mg of dihydrate (obtained as described in Example 2.1 below) were suspended in 2 mL of ethyl acetate. The suspension was stirred for one week at ambient conditions (approx. 20°C and 1013 mbar) in a closed vessel. The vessel was opened after this time period and the suspension was allowed to dry completely at ambient conditions.

The obtained crystals of monohydrate were isolated and analyzed by X-ray powder diffraction (XRPD).

### I.2 Properties of the monohydrate of A4356716H of formula (1)

The below mentioned properties are beneficial during the manufacturing of a agrochemical composition due to (mechanical) stability.

### I.2.1 Mechanical stability

The crystalline monohydrate of A4356716H did not change substantially under the influence of mechanical stress (ball milling). In particular, no loss of water was observed.

### Example II - Dihydrate of 4-amino-3-chloro-5-fluoro-6-(7-fluoro-1H-indol-6-yl)picolinic acid

### II.1 Preparation of the dihydrate of A4356716H of formula (1)

### Example 2.1: Process for the production of dihydrate

A solution of methyl 4-amino-3-chloro-5-fluoro-6-(7-fluoro-1H-indol-6-yl)pyridine-2-carboxylate (2.20 g, 6.51 mmol) and lithium hydroxide (0.22 g, 9.12 mmol) in 22 mL of tetrahydrofuran (THF) and 49 mL of water was heated at 65 °C for 4.5 h, then stood at room temperature (approx. 20°C) overnight. Subsequently, THF was removed by evaporation and the remaining aqueous solution acidified to pH 2 with 2M HCl. The resulting precipitate was isolated by filtration to yield 1.77 g of 4-amino-3-chloro-5-fluoro-6-(7-fluoro-1H-indol-6-yl)pyridine-2-carboxylic acid in the form of the dihydrate. Upon standing, a further 0.40 g of dihydrate were obtained by filtration.

The obtained crystals of the dihydrate were isolated and analyzed by X-ray powder diffraction (XRPD).

### II.2 Stability: Slurry / stirring experiments

In each experiment, about 100 mg of the crystalline dihydrate (obtained as described in Example 2.1 below) were suspended in 2 mL of the respective solvent and stirred at 25°C. After one week, each suspension was filtered and the respective residue obtained dried at ambient temperature and ambient humidity. The resulting material was analysed. The obtained solids were isolated and analyzed by X-ray powder diffraction (XRPD). The respective result is stated in column "Result" in Table S.

**Table S: Results of slurry / stirring experiments**

| **Example** | **Solvent/Solvent Mixture** | **Result** |
|---|---|---|
| S1 | Ethanol / Water (1 : 1) | Dihydrate |
| S2 | Ethyl acetate | Monohydrate |
| S3 | Toluene | Mixture of Monohydrate and Dihydrate |

### Example III - Nonstoichiometric hydrate of 4-amino-3-chloro-5-fluoro-6-(7-fluoro-1H-indol-6-yl)picolinic acid

### III.1 Preparation of the nonstoichiometric hydrate form of A4356716H of formula (1)

### Example 3.1: Process for the production of nonstoichiometric hydrate form

500 mg of dihydrate (obtained as described in Example 2.1 above) were dissolved in 40 mL of tetrahydrofuran (THF). The clear solution was stored in a refrigerator (approx. 4 °C) until the solvent had evaporated completely. As a result, 4-amino-3-chloro-5-fluoro-6-(7-fluoro-1H-indol-6-yl)pyridine-2-carboxylic acid in the form of the nonstoichiometric hydrate form was obtained.

The nonstoichiometric hydrate form was also obtained when repeating the above process and storing the clear solution in a freezer (approx. -18 °C) until the solvent had evaporated completely.

The obtained crystals of the nonstoichiometric hydrate were isolated and analyzed by X-ray powder diffraction (XRPD).

## Claims

1. Crystalline monohydrate of the compound of formula (1) 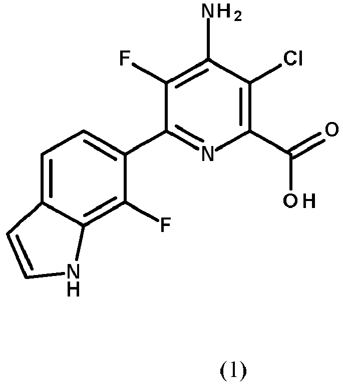 which in a X-ray powder diffractogram at 25°C and Cu-Kα 1 radiation displays at least the following reflections, each quoted as 2θ value ± 0.2°: 24.8; 26.2 and 31.2.

2. The monohydrate of the compound of claim 1, which in a X-ray powder diffractogram at 25°C and Cu-Kα 1 radiation displays at least the following reflections, each quoted as 2θ value ± 0.2°: 24.8; 26.2; 31.2; 34.1 and 16.5.

3. The monohydrate of the compound of claim 1, which in a X-ray powder diffractogram at 25°C and Cu-Kα 1 radiation displays at least the following reflections, each quoted as 2θ value ± 0.2°: 24.8; 26.2; 31.2; 34.1; 16.5; 34.4 and 7.9.

4. The monohydrate of the compound of claim 1, which in a X-ray powder diffractogram at 25°C and Cu-Kα 1 radiation displays at least the following reflections, each quoted as 2θ value ± 0.2°: 24.8; 26.2; 31.2; 34.1; 16.5; 34.4; 7.9; 12.1 and 39.3.

5. A process for the production of monohydrate according to any of claims 1 to 4, comprising the following steps:
a) suspending the compound of formula (1) in a solvent or solvent mixture selected from the group consisting of
(i) of aliphatic esters with a total of 3 to 6 carbon atoms;
or
(ii) isopropanol;
b) stirring the suspension of step a) at a temperature in the range of from about 15°C to about 30°C for about 24 to 240 hours in a closed vessel;
c) evaporating the solvent or solvent mixture from the mixture obtained in step b) at a temperature in the range of from about 15°C to about 30°C.

6. The process according to claim 5, wherein the solvent or solvent mixture of step a) is selected from the group consisting of the C₁-C₄-esters of acetic acid, preferably selected from the group consisting of methyl acetate, ethyl acetate, n-propyl acetate or n-butyl acetate.

7. The process according to claim 5, wherein the solvent is isopropanol.

8. The process according to any one of claims 5 to 7, wherein in step b) the suspension obtained in step a) is stirred at a temperature in the range of about 18°C to about 25°C for about 120 to 200 hours.

9. A plant protection agent containing the monohydrate of formula (1) according to any of claims 1 to 4.

10. The plant protection agent according to claim 9, which further comprises one or more further constituents selected from the group of agriculturally acceptable additive(s) customary for the formulation of plant protection agents, further herbicides, insecticides, acaricides, fungicides, safeners and/or plant growth regulator.

11. The plant protection agent according to any of claims 9 or 10, wherein said plant protection agent contains water.

12. The plant protection agent according to any of claims 9 to 11, wherein said plant protection agent is in the form of a suspension concentrate.

13. Use of the monohydrate of the compound of formula (1) according to any of claims 1 to 4 or of a plant protection agent according to any of claims 9 to 11 for the production of a formulation in the form of a suspension concentrate.

14. Use of the monohydrate of the compound of formula (1) according to any of claims 1 to 4 or of a plant protection agent according to any of claims 9 to 12 for combatting undesired plant growth, preferably for combatting undesired plant growth in crops of useful plants.

15. Method for combating undesired plant growth, wherein the monohydrate according to any of claims 1 to 4 or a plant protection agent according to any of claims 9 to 12 is applied to plants or the site of the undesired vegetation.
